# EUROPEAN PATENT SPECIFICATION

(11) **EP 0 573 544 B1**
(45) Date of publication and mention of the grant of the patent: **29.05.2002**
(21) Application number: 92906791.6
(22) Date of filing: 13.02.1992
(51) Int. Cl.: C12N 15/12, C12N 1/21, C07K 14/575, A61K 38/22, C07K 16/26, G01N 33/53, G01N 33/50, C12N 15/63

(54) **INTESTINAL TREFOIL PROTEINS**
INTESTINALE KLEEBLATT-PROTEINE
PROTEINES TRIFOLIEES INTESTINALES

(30) Priority: 14.02.1991 US 655965
(43) Date of publication of application: 15.12.1993
(73) Proprietor: THE GENERAL HOSPITAL CORPORATION, Charlestown, MA 02129 (US)
(72) Inventor: PODOLSKY, Daniel, K., Dr., Wellesley Hills, MA 02181 (US)
(74) Representative: Wallace, Sheila Jane
(86) International application number: US9201200
(87) International publication number: WO9214837

(56) References cited:
- PROCEEDINGS OF THE NATIONAL ACADEMY OF SCIENCES OF USA. vol. 88, no. 24 , 15 December 1991 , WASHINGTON US pages 11017 - 11021 S. SUEMORI ET AL. 'Identification and characterization of rat intestinal trefoil factor: Tissue- and cell-specific member of the trefoil protein family'
- SAMBROOK et al., "Molecular Cloning, A Laboratory Manual", 2nd Edition, published 1989 by Cold Spring Harbor Laboratory Press, (NY), pp. 8.3 to 8.52 and 12.1 to 12.29.
- SAMBROOK et al., "Molecular Cloning, A Laboratory Manual", published 1989 by Cold Spring Harbor Laboratory, (NY), pp. 18.1 to 18.29.
- FRANK ROLE, "Remmington's Pharmaceutical Sciences", 18th edition, published 1990 by Mack Publishing Co., (Easton, PA.), pp. 1389-1404.
- Gastroenterology, Volume 100, No. 5, Part 2, 19-22 May 1991, S. SUEMORI et al., "Identification and Molecular Cloning of a New Intestinal Trefoil Growth Factor", Abstract No. A550, see the Abstract.
- C. TOMASETTO ET AL.: "hSP, the domain-duplicated homolog of pS2 protein, is co-expressed with pS2 in stomach but not in breast carcinoma", THE EMBO JOURNAL, , , vol. 9, no. 2, pages 407 to 414

### SEQUENCE LISTING

**(1) GENERAL INFORMATION:**
   **(i) APPLICANT:** Podolsky, Daniel K.
   **(ii) TITLE OF INVENTION:** INTESTINAL TREFOIL FACTORS
   **(iii) NUMBER OF SEQUENCES:** 3
   **(iv) CORRESPONDENCE ADDRESS:**
      **(A) ADDRESSEE:** Fish & Richardson
      **(B) STREET:** 225 Franklin Street
      **(C) CITY:** Boston
      **(D) STATE:** Massachusetts
      **(E) COUNTRY:** U.S.A.
      **(F) ZIP:** 02110-2804
   **(v) COMPUTER READABLE FORM:**
      **(A) MEDIUM TYPE:** 3.5" Diskette, 1.44 Mb
      **(B) COMPUTER:** IBM PS/2 Model 50Z or 55SX
      **(C) OPERATING SYSTEM:** IBM P.C. DOS (Version 3.30)
      **(D) SOFTWARE:** WordPerfect (Version 5.0)
   **(vi) CURRENT APPLICATION DATA:**
      **(A) APPLICATION NUMBER:**
      **(B) FILING DATE:**
      **(C) CLASSIFICATION:**
   **(vii) PRIOR APPLICATION DATA:**
      **(A) APPLICATION NUMBER:**
      **(B) FILING DATE:**
   **(viii) ATTORNEY/AGENT INFORMATION:**
      **(A) NAME:** Clark, Paul T.
      **(B) REGISTRATION NUMBER:** 30,162
      **(C) REFERENCE/DOCKET NUMBER:** 00786/066001
   **(ix) TELECOMMUNICATION INFORMATION:**
      **(A) TELEPHONE:** (617) 542-5070
      **(B) TELEFAX:** (617) 542-8906
      **(C) TELEX:** 200154
**(2) INFORMATION FOR SEQUENCE IDENTIFICATION NUMBER:** 1:
   **(i) SEQUENCE CHARACTERISTICS:**
      **(A) LENGTH:** 431
      **(B) TYPE:** nucleic acid
      **(C) STRANDEDNESS:** single
      **(D) TOPOLOGY:** linear
   **(xi) SEQUENCE DESCRIPTION:** SEQ ID NO: 1: 
GAAGTTTGCG TGCTGCC 17

ATG GAG ACC AGA GCC TTC TGG ATA ACC CTG CTG CTG GTC CTG GTT GCT 65
Met Glu Thr Arg Ala Phe Trp Ile Thr Leu Leu Leu Val Leu Val Ala
1               5                   10                  15

GGG TCC TCC TGC AAA GCC CAG GAA TTT GTT GGC CTA TCT CCA AGC CAA 113
Gly Ser Ser Cys Lys Ala Gln Glu Phe Val Gly Leu Ser Pro Ser Gln
            20                  25                  30

TGT ATG GCG CCA ACA AAT GTC AGG GTG GAC TGT AAC TAC CCC ACT GTC 161
Cys Met Ala Pro Thr Asn Val Arg Val Asp Cys Asn Tyr Pro Thr Val
        35                  40                  45

ACA TCA GAG CAG TGT AAC AAC CGT GGT TGC TGT TTT GAC TCC AGC ATC 209
Thr Ser Glu Gln Cys Asn Asn Arg Gly Cys Cys Phe Asp Ser Ser Ile
    50                  55                  60

CCA AAT GTG CCC TGG TGC TTC AAA CCT CTG CAA GAG ACA GAA TGT ACA 257
Pro Asn Val Pro Trp Cys Phe Lys Pro Leu Gln Glu Thr Glu Cys Thr
65                  70                  75                  80

TTT 260
Phe

TGAAGCTGTC CAGGCTCCAG GAAGGGAGCT CCACACCCTG GACTCTTGCT GATGGTAGTG 320

GCCCAGGGTA ACACTCACCC CTGATCTGCT CCCTCGCGCC GGCCAATATA GGAGCTGGGA 380

GTCCAGAAGA ATAAAGACCT TACAGTCAGC ACAAGGCTGT TCTAATTGCG G 431
**(2) INFORMATION FOR SEQUENCE IDENTIFICATION NUMBER:** 2:
   **(i) SEQUENCE CHARACTERISTICS:**
      **(A) LENGTH:** 81
      **(B) TYPE:** amino acid
      **(C) STRANDEDNESS:** N/A
      **(D) TOPOLOGY:** N/A
   **(xi) SEQUENCE DESCRIPTION:** SEQ ID NO: 2: 
Met Glu Thr Arg Ala Phe Trp Ile Thr Leu Leu Leu Val Leu Val Ala
                5                   10                  15

Gly Ser Ser Cys Lys Ala Gln Glu Phe Val Gly Leu Ser Pro Ser Gln
            20                  25                  30

Cys Met Ala Pro Thr Asn Val Arg Val Asp Cys Asn Tyr Pro Thr Val
        35                  40                  45

Thr Ser Glu Gln Cys Asn Asn Arg Gly Cys Cys Phe Asp Ser Ser Ile
    50                  55                  60

Pro Asn Val Pro Trp Cys Phe Lys Pro Leu Gln Glu Thr Glu Cys Thr
65                  70                  75                  80

Phe
**(2) INFORMATION FOR SEQUENCE IDENTIFICATION NUMBER:** 3:
   **(i) SEQUENCE CHARACTERISTICS:**
      **(A) LENGTH:** 403
      **(B) TYPE:** nucleic acid
      **(C) STRANDEDNESS:** single
      **(D) TOPOLOGY:** linear
   **(xi) SEQUENCE DESCRIPTION:** SEQ ID NO: 3:

G                                                                 1

ATG CTG GGG CTG GTC CTG GCC TTG CTG TCC TCC AGC TCT GCT GAG GAG      49
Met Leu Gly Leu Val Leu Ala Leu Leu Ser Ser Ser Ser Ala Glu Glu
1               5                   10                  15

TAC GTG GGC CTG TCT GCA AAC CAG TGT GCC GTG CCG GCC AAG GAC AGG      97
Tyr Val Gly Leu Ser Ala Asn Gln Cys Ala Val Pro Ala Lys Asp Arg
            20                  25                  30

GTG GAC TGC GGC TAC CCC CAT GTC ACC CCC AAG GAG TGC AAC AAC CGG      145
Val Asp Cys Gly Tyr Pro His Val Thr Pro Lys Glu Cys Asn Asn Arg
        35                  40                  45

GGC TGC TGC TTT GAC TCC AGG ATC CCT GGA GTG CCT TGG TGT TTC AAG      193
Gly Cys Cys Phe Asp Ser Arg Ile Pro Gly Val Pro Trp Cys Phe Lys
    50                  55                  60

CCC CTG ACT AGG AAG ACA GAA TGC ACC TTC 223
Pro Leu Thr Arg Lys Thr Glu Cys Thr Phe
65                  70

TGAGGCACCT CCAGCTGCCC CTGGGATGCA GGCTGAGCAC CCTTGCCCGG CTGTGATTGC 283

TGCCAGGCAC TGTTCATCTC AGTTTTTCTG TCCCTTTGCT CCCGGCAAGC TTTCTGCTGA 343

AAGTTCATAT CTGGAGCCTG ATGTCTTAAC GAATAAAGGT CCCATGCTCC ACCCGAAAAA 403

## Description

This invention relates to peptides useful for treatment of disorders of the digestive system, in particular, intestinal trefoil factor.

Jørgensen et al. (Regulatory Peptides 3:231, 1982) describe a porcine pancreatic peptide, pancreatic spasmolytic peptide (PSP) - PSP was found to inhibit "gastrointestinal motility and gastric acid secretion in laboratory animal after parenteral as well as oral administration." It was suggested that "if the results in animal experiments can be confirmed in man, PSP may possess a potential utility in treatment of gastroduodenal ulcer diseases.

Tomasetto, et al *(EMBO J.,* 1990, 9:407-414) discloses the sequence of a cDNA clone of PSP, together with a sequence of a human counterpart (hSP) and a mouse counterpart (mSP).

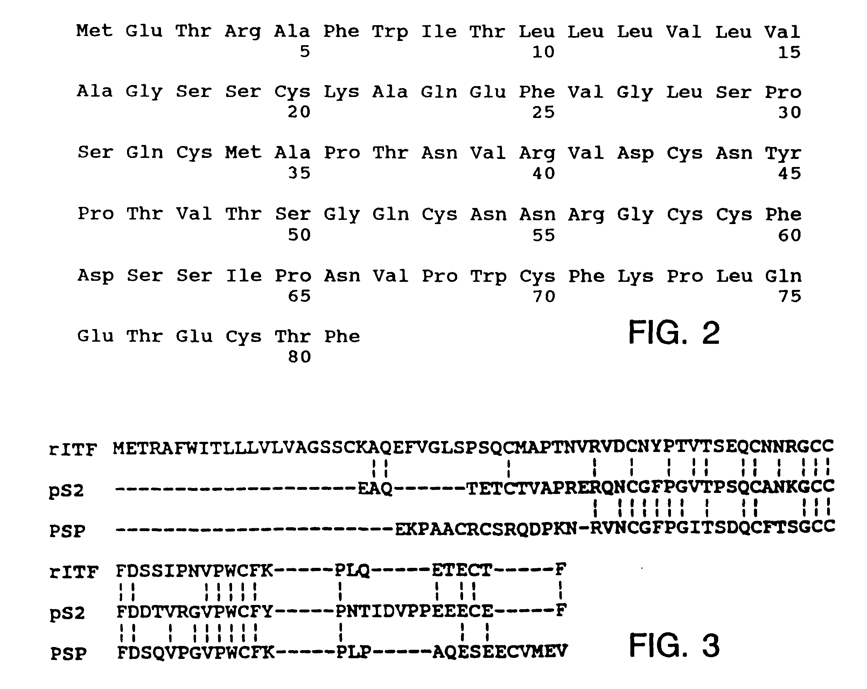

The term "intestinal trefoil factor" ("ITF") includes any protein which is substantially homologous to rat intestinal trefoil factor (Fig. 2, SEQ ID NO 2) and which is expressed in the large intestine, small intestine, or colon to a greater extent than it is expressed in tissues other than the small intestine, large intestine, or colon. Also included are: allelic variations; natural mutants; induced mutants; proteins encoded by DNA that hybridizes under high or low stringency conditions to ITF encoding nucleic acids retrieved from naturally occurring material; and polypeptides or proteins retrieved by antisera to ITF, especially by antisera to the active site or binding domain of ITF. The term also includes other chimeric polypeptides that include an ITF.

The term ITF also includes analogs of naturally occurring ITF polypeptides. Analogs can differ from naturally occurring ITF by amino acid sequence differences or by modifications that do not affect sequence, or by both. Analogs within the scope of the invention will generally exhibit at least 70%, more preferably 80%, more preferably 90%, and most preferably 95% or even 99%, homology with all or part of a naturally occurring ITF sequence. The length of comparison sequences will generally be at least about 8 amino acid residues, usually at least 20 amino acid residues, more usually at least 24 amino acid-residues, typically at least 28 amino acid residues, and preferably more than 35 amino acid residues. Modifications include *in vivo,* or *in vitro* chemical derivatization of polypeptides, e.g., acetylation, or carboxylation. Also included are modifications of glycosylation, e.g., those made by modifying the glycosylation patterns of a polypeptide during its synthesis and processing or in further processing steps, e.g., by exposing the polypeptide to enzymes that affect glycosylation derived from cells that normally provide such processing, e.g., mammalian glycosylation enzymes. Also embraced are versions of the same primary amino acid sequence that have phosphorylated amino acid residues, e.g., phosphotyrosine, phosphoserine, or phosphothreonine. Analogs can differ from naturally occuring ITF by alterations of their primary sequence. These include genetic variants, both natural and induced. Induced mutants may be derived by various techniques, including random mutagenesis of the encoding nucleic acids using irradiation or exposure to ethanemethylsulfate (EMS), or may incorporate changes produced by site-specific mutagenesis or other techniques of molecular biology. See, Sambrook, Fritsch and Maniatis (1989), Molecular Cloning: A Laboratory Manual (2d ed.), CSH Press. Also included are analogs that include residues other than naturally occurring L-amino acids., e,g., D-amino acids or non-naturally occurring or synthetic amino acids, e.g., β or γ amino acids.

In addition to substantially full-length polypeptides, the term ITF, as used herein, includes biologically active fragments of the polypeptides. As used herein, the term "fragment", as applied to a polypeptide, will ordinarily be at least about 10 contiguous amino acids, typically at least about 20 contiguous amino acids, more typically at least about 30 contiguous amino acids, usually at least about 40 contiguous amino acids, preferably at least about 50 contiguous amino acids, and most preferably at least about 60 to 80 or more contiguous amino acids in length. Fragments of ITF can be generated by methods known to those skilled in the art. The ability of a candidate fragment to exhibit a biological activity of ITF can be assessed by methods known to those skilled in the art. Also included in the term are biologically active ITF polypeptides containing amino acids that are normally removed during protein processing, including additional amino acids that are not required for the biological activity of the polypeptide, or including additional amino acids that result from alternative mRNA splicing or alternative protein processing events.

An ITF polypeptide, fragment, or analog is biologically active if it exhibits a biological activity of a naturally occurring ITF, e.g., the ability to alter gastrointestinal motility in a mammal.

In a first aspect, the invention features a purified nucleic acid encoding an intestinal trefoil factor (ITF) comprising a polypeptide that is at least 70% homologous to SEQ ID NO: 2.

In preferred embodiments, the intestinal trefoil factor is mammalian intestinal trefoil factor, preferably human, rat, bovine, or porcine intestinal trefoil factor. In another preferred embodiment, a vector is provided which comprises nucleic acid encoding such an intestinal trefoil, factor.

In a second alternative aspect, the invention features a cell that includes a vector encoding an intestinal trefoil factor comprising a polypeptide that is at least 70% homologous to SEQ ID NO: 2.

In a third related aspect, the invention features a substantially pure intestinal trefoil factor comprising a polypeptide that is at least 70% homologous to SEQ ID NO:2. In a preferred embodiment, the polypeptide is detectably labelled. In a fourth related aspect, the invention features a therapeutic composition that includes such an intestinal trefoil factor and a pharmacologically acceptable carrier.

In a fifth alternative aspect, the invention features a monoclonal antibody which forms an immune complex with such an intestinal trefoil factor. In a preferred embodiment, the monoclonal antibody is detectably labelled.

In a sixth related aspect, the invention features a method for detecting human intestinal trefoil factor in a human patient. The method includes the steps of contacting a biological sample obtained from the patient with a monoclonal antibody which forms an immune complex with intestinal trefoil factor, and detecting immune complexes formed with the monoclonal antibody. In preferred embodiments the biological sample is an intestinal mucosal scraping, or serum.

In a seventh alternative aspect, the invention features a method for detecting binding sites for intestinal trefoil factor comprising a polypeptide which is at least 70% homologous to SEQ ID NO:2 in a biological sample obtained from the patient. The sample is contacted with the substantially pure factor, and detecting the factor bound to the biological sample is an indication of the presence of the binding sites in the sample. By "binding sites", as used herein, is meant antibody or receptor that binds to an intestinal trefoil factor protein, factor, or analog.

The detection or quantitation of binding sites may be useful in reflecting abnormalities of the gastrointestinal tract.

In preferred embodiments, the intestinal trefoil factor is human, porcine, or bovine trefoil factor.

A further eighth aspect of this invention provides for a method for detecting binding sites for an intestinal trefoil factor comprising a polypeptide which is at least 70% homologous to SEQ ID NO: 2 in a biological sample obtained from a patient comprising contacting said sample with a substantially pure intestinal trefoil factor according to the third aspect of the invention and detecting said factor bound to said biological sample as an indication of the presence of said binding sites in said sample.

In a ninth related aspect of the invention, we provide isolated DNA comprising a sequence encoding an intestinal trefoil factor comprising a polypeptide that is at least 70% homologous to SEQ ID NO: 2.

We further provide, in accordance with a further alternative aspect of the invention, a vector comprising nucleic acid according to the first aspect of the invention, or isolated DNA according to the ninth aspect of the invention. Furthermore, we provide a polypeptide according to the third aspect of the present invention, for the manufacture of a medicament for use in treating digestive disorders.

In accordance with yet further aspects of the invention, we provide a purified nucleic acid comprising the sequence of SEQ ID NO: 1 and a substantially pure polypeptide comprising the sequence of SEQ ID NO: 2.

There is further provided, in accordance with a further aspect of the invention a purified nucleic acid encoding a human intestinal trefoil factor, the nucleic acid producible by a method comprising the steps of: providing a human intestinal cDNA library; providing a pair of oligonucleotide primers having sequences of a trefoil encoding region of rat intestinal trefoil factor nucleic acid, the rat nucleic acid having a sequence SEQ ID: 1; screening the cDNA library for nucleic acid sequences which are amplified by the oligonucleotide pairs in a polymerase chain reaction; and selecting from the cDNA library those sequences which correspond to the amplified nucleic acid sequences and which show a pattern of expression in the small and large intestine, but not in stomach or liver.

It will thus be seen that the invention extends to nucleic acid sequences, and purified preparations thereof, that encode the ITF polypeptides described herein. We also describe antibodies, preferably monoclonal antibodies, that bind specifically to ITF polypeptides.

As used herein, the term "substantially pure" describes a compound, e.g., a nucleic acid, a protein, or a polypeptide, e.g., an ITF protein or polypeptide, that is substantially free from the components that naturally accompany it. Typically, a compound is substantially pure when at least 60%, more preferrably at least 75%, more preferrably at least 90%, and most preferrably at least 99%, of the total material (by volume, by wet or dry weight, or by mole per cent or mole fraction) in a sample is the compound of interest. Purity can be measured by any appropriate method, e.g., in the case of polypeptides by column chromatography, polyacrylamide gel electrophoresis, or HPLC analysis.

By "isolated DNA" is meant that the given DNA is free of the genes which, in the naturally-occurring genome of the organism from which the given DNA of the invention is derived, flank the given DNA. The term "isolated DNA" thus encompasses, for example, cDNA, cloned genomic DNA, and synthetic DNA. A "purified nucleic acid", as used herein, refers to a nucleic acid sequence that is substantially free of other macromolecules (e.g., other nucleic acids and proteins) with which it naturally occurs within a cell. In preferred embodiments, less than 40% (and more preferably less than 25%) of the purified nucleic acid preparation consists of such other macromolecules.

"Homologous", as used herein, refers to the subunit sequence similarity between two polymeric molecules, e.g., between two nucleic acid molecules, e.g., two DNA molecules, or two polypeptide molecules. When a subunit position in both of the two molecules is occupied by the same monomeric subunit, e.g., if a position in each of two DNA molecules is occupied by adenine, then they are homologous at that position. The homology between two sequences is a direct function of the number of matching or homologous positions, e.g., if half, e.g., 5 of 10, of the positions in two compound sequences are homologous then the two sequences are 50% homologous, if 90% of the positions, e.g., 9 of 10, are matched or homologous the two sequences share 90% homology. By way of example, the DNA sequences 3'ATTGCC'5 and 3'TATGGC'5 share 50% homology. By "substantially homologous" is meant largely but not wholly homologous.

Our ITF proteins are resistant to destruction in the digestive tract, and can be used for treatment of peptic ulcer diseases, inflammatory bowel diseases, and for protection of the intestinal tract from injury caused by bacterial infection, radiation injury or other insults. An ITF protein, fragment, or analog can also be used to treat neoplastic cancer.

Other features and advantages will be apparent from the following description of the preferred embodiments.

The drawings will first be briefly described.
Figure 1 is a depiction of the nucleotide sequence of rat trefoil factor (SEQ ID NO: 1).
Figure 2 is a depiction of the deduced amino acid sequence of rat trefoil factor (SEQ ID NO: 2).
Figure 3 is a depiction of the amino acid sequences of rat trefoil factor, pS2 protein, and pancreatic spasmolytic polypeptide. The sequences are aligned so as to illustrate the amino acid sequence homology between the proteins. Dashes (-) indicate the insertion of spaces which improve alignment. Bars ( ) indicate sequence identities.
Figure 4 depicts the disulfide bond structure proposed for pS2 (panel A) and PSP (panel B);
Figure 5 is a depiction of the proposed disulfide bond structure of rat intestinal trefoil factor.
Figure 6 is a depiction of the nucleotide sequence of the human intestinal trefoil factor cDNA and the corresponding deduced amino acid sequence (SEQ ID NO: 3).

### Purification and cloning of rITF

An inhibitor of soft agar colony formation by human breast carcinoma-derived BT-20 cells (ATTC HTB79) was isolated from cytology-positive human malignant effusions (Podolsky *et al.,* Cancer Res. 48:418, 1988). The factor also inhibited soft agar colony formation by human colon carcinoma-derived HCT15 cells (ATTC-CCL225). Inhibition was not observed for polyoma and murine sarcoma virus transformed rodent fibroblast lines. The isolated factor (transformed cell-growth inhibiting factor or TGIF) had an apparent molecular weight of 110,000 kD and appeared to consist of two 55,000 kD subunits linked by sulfhydryl bonds.

The purified protein was partially sequenced. The sequence from the amino terminal 14 amino acids was used to produce a set of degenerate oligonucleotide probes for screening of a rat intestinal epithelial cell cDNA library.

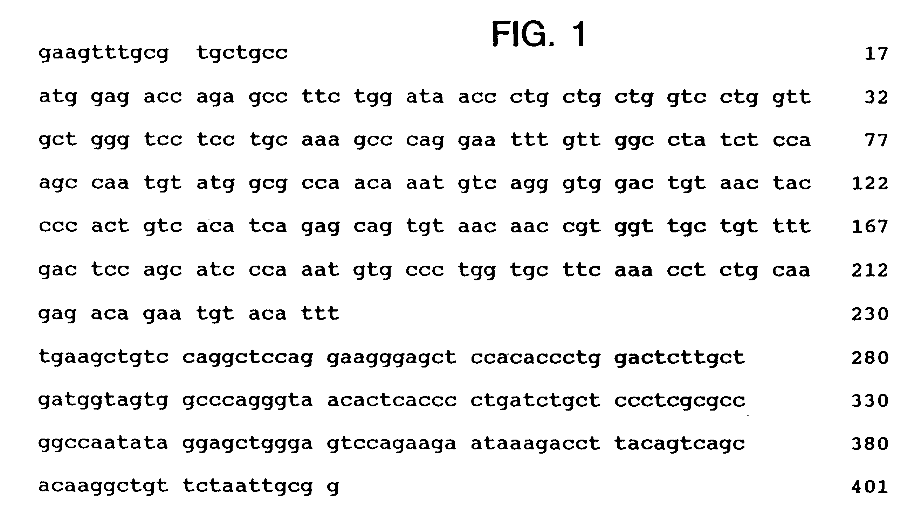

A rat intestinal cDNA library (Lambda ZAP° II, Stratagene, La Jolla, CA) was produced by standard techniques (Ausubel et al., eds., Current *Protocols* in *Molecular Biology,* John Wiley & Sons, New York, 1989) using cells purified by the method of Weisner (*J. Biol* Chem. 248:2536, 1973). Screening of the cDNA library with the fully degenerate oligonucleotide probe described above resulted in the selection of 21 clones. One of the clones (T3411) included a core sequence which encoded a single open reading frame. The nucleotide sequence of the open reading frame and flanking DNA is presented in Fig. 1 (SEQ ID NO 1). The insert present in T3411 was nick translated (Ausubel et al., *supra)* to produce a radioactively labelled probe for Northern blot analysis of rat poly(A)⁺ RNA. Northern analysis demonstrated that RNA corresponding to the cloned cDNA fragment was expressed in small intestine, large intestine, and kidney; no expression was detected in the lung, spleen, heart, testes, muscle, stomach, pancreas, or liver. In the tissues in which the RNA was expressed, the level was comparable to that of actin.

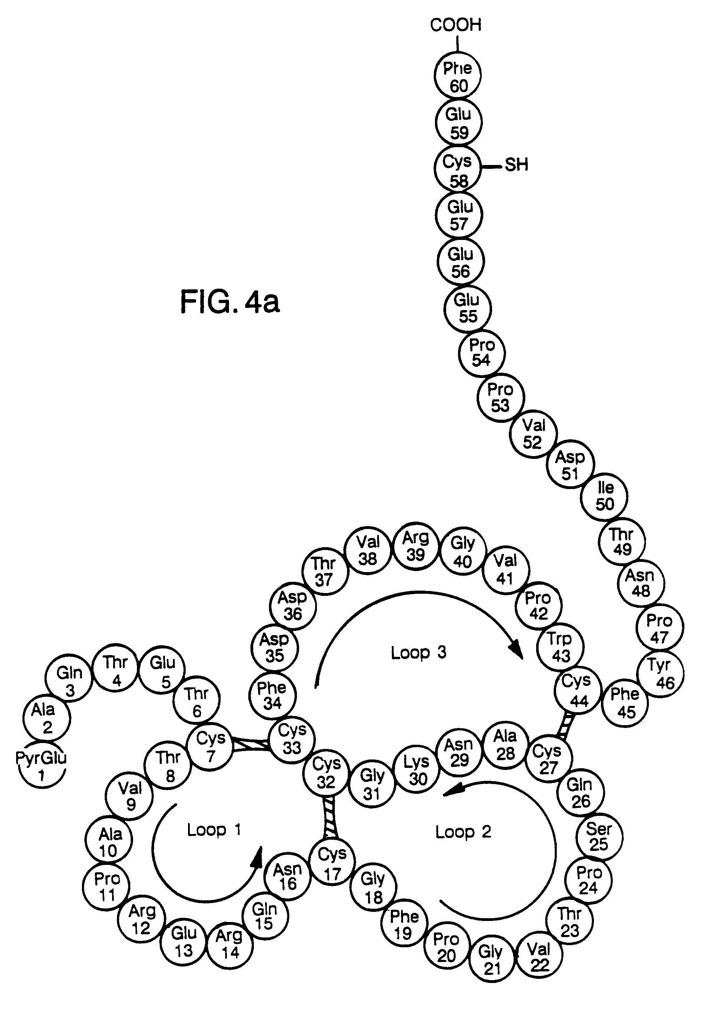

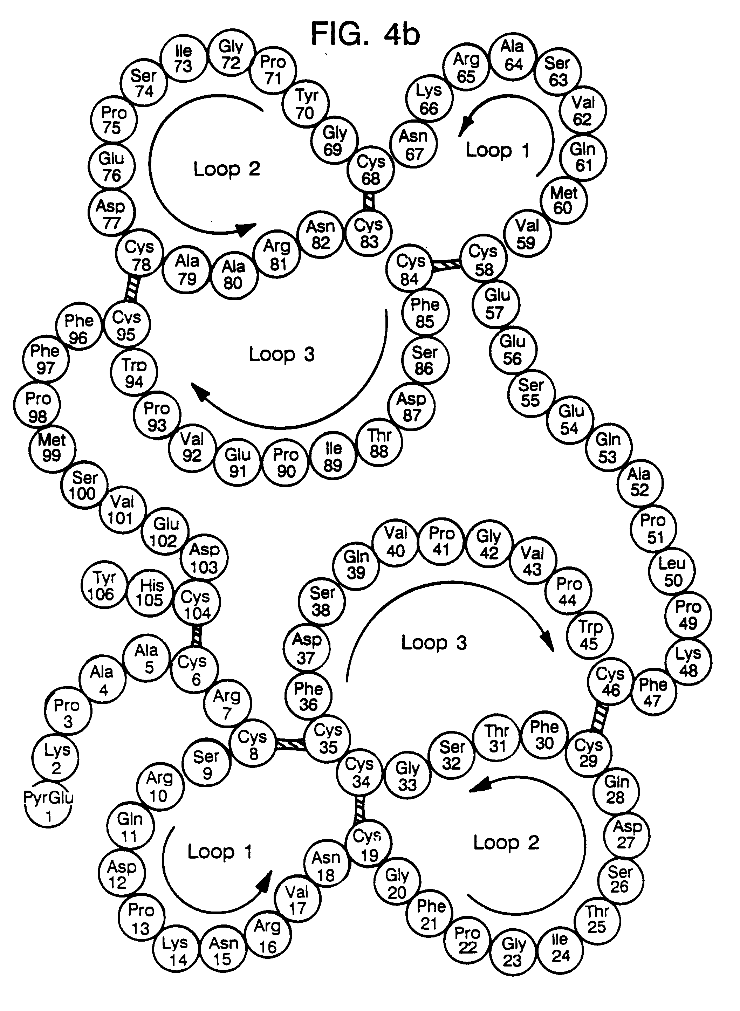

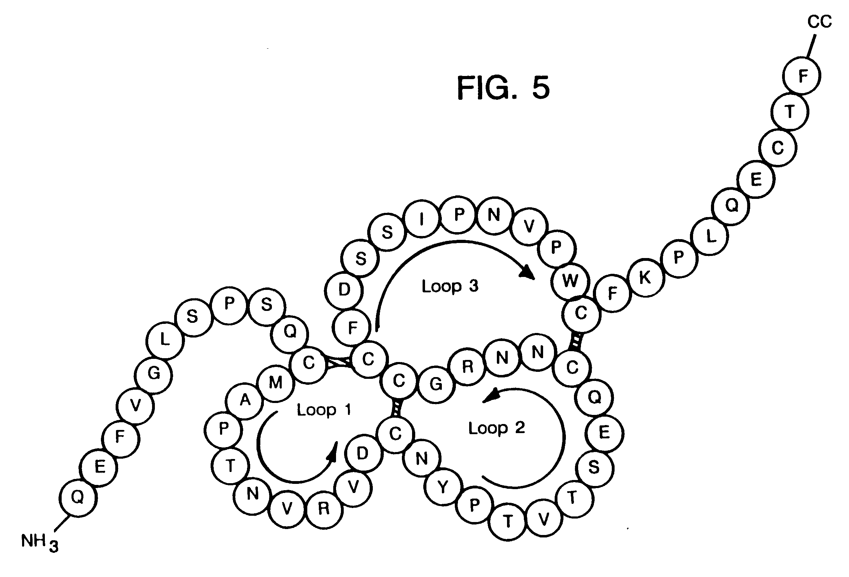

The open reading frame of clone T3411 encoded an 81 amino acid peptide (Fig. 2; SEQ ID NO 2). Comparison of the sequence of the encoded peptide, referred to as rat intestinal trefoil factor (rITF), to the sequence of proteins in the Genebank database revealed significant homology to human breast cancer associated peptide (pS2; Jakowlev et al., Nucleic *Acids* Res. 12:2861, 1984) and porcine pancreatic spasmolytic peptide (PSP; Thim et al., *Biochem. Biophys. Acta* 827:410, 1985). Fig. 3 illustrates the homology between rITF, PSP and pS2. Porcine pancreatic spasmolytic factor (PSP) and pS2 are both thought to fold into a characteristic structure referred to as a trefoil. A trefoil structure consists of three loops formed by three disulfide bonds, pS2 is thought to include one trefoil (Fig. 4A), and PSP is thought to include two trefoils (Fig. 4B). The region of rITF (nucleotide 114 to nucleotide 230 which encodes cys to phe) which is most similar to PSP and pS2 includes six cysteines a1l of which are in the same position as the cysteines which make up the trefoil in pS2 (Fig. 3). Five of these six cysteines are in the same position as the cysteines which form the amino terminal trefoil of PSP (Fig. 3). Fig. 5 depicts the proposed disulfide bond configuration of rITF.

Based on homology to PSP and pS2 (Mori et al., *Biochem. Biophys. Res. Comm. 155:366,* 1988; Jakowlew et al., *Nucleic Acids Res.* 12:2861, 1984), rITF includes a presumptive pro- sequence (met¹ to ala²²) in which 12 of 22 amino acids have hydrophobic side chains.

### Production of Anti-rITF Antibodies

A peptide corresponding to the carboxy-terminal 21 amino acids of rITF was synthesized and coupled to bovine serum albumin (BSA). This conjugate (and the unconjugated peptide) was used to raise polyclonal antibodies in rabbits. All procedures were standard protocols such as those described in Ausubel et al. *(supra).* The anti-rITF antibodies were used in an indirect immunoflouresce assay for visualization of rITF in rat tissues. Cryosections of rat tissues were prepared using standard techniques, and fluorescein labelled goat anti-rabbit monoclonal antibody (labelled antibodies are available from such suppliers Kirkegaard and Perry Laboratories, Gaithersberg, MD; and Bioproducts for Science, In., Indianapolis, IN) was used to detect binding of rabbit anti-rITF antibodies. By this analysis rITF appears to be present in the globlet cells of the small intestine but not in the stomach or the pancreas.

### Cloning of Human Intestinal Trefoil Factor

DNA encoding the rat intestinal trefoil factor can be used to identify a cDNA clone encoding the human intestinal trefoil factor (hITF). This can be accomplished by screening a human colon cDNA library with a probe derived from rITF or with a probe derived from part of the hITF gene. The latter probe can be obtained from a human colon or intestinal cDNA using the polymerase chain reaction to isolate a part of the hITF gene. This probe can then serve as a specific probe for the identification of clones encoding all of the hITF gene.

### Construction of a cDNA Library.

A human colon or intestinal cDNA library in λgt10 or λgtll, or some other suitable vector is useful for isolation of hITF. Such libraries may be purchased (Clontech Laboratories, Palo Alto, CA: HLI034a, HLI0346b). Alternatively, a library can be produced using mucosal scrapings from human colon or intestine. Briefly, total RNA is isolated from the tissue essentially as described by Chirgwin et al. *(Biochemistry* 18:5294, 1979; see also Ausubel et al., *supra).* An oligo (dT) column is then used to isolate poly(A)⁺ RNA by the method of Aviv et al. (J. *Mol. Biol.* 134:743, 1972; see also Ausubel et al., *supra).* Double-stranded cDNA is then produced by reverse transcription using oligo (dT)_{12.18} or random hexamer primers (or both). RNAse H and E. *coli* DNA poll are then used to replace the RNA strand with a second DNA strand. In a subsequent step E. *coli* DNA ligase and T4 DNA polymerase are used to close gaps in the second DNA strand and create blunt ends. Generally, the cDNA created is next methylated with EcoRI methylase and EcoRI linkers are added (other linkers can be used depending on the vector to be used). In subsequent steps the excess linkers are removed by restriction digestion and the cDNA fragments are inserted into the desired vector. See Ausubel et al., *supra* and Sambrook et al. *(Molecular Cloning: A Laboratory Manual,* Cold Spring Harbor Laboratory, Cold Spring Harbor, NY, 1990) for detailed protocols. Useful vectors include: λgtll, λgtlO, Lambda ZAP® II vector, Lambda Uni-ZAP™ XR vector, all available from Stratagene (La Jolla, CA).

The cDNA library must be packaged into phage; this is most readily accomplished by use of a commercial in *vitro* packaging kit, e.g., Gigapack® II Gold or Gigapack® II Plus (Stratagene, La Jolla, CA). See Ausubel et al. *(supra)* for packaging protocols and suitable host strains. The library is preferably amplified soon after packaging; this step generates sufficient clones for multiple screening of the library. See Ausubel et al. *supra* or Sambrook et al. *supra* for details of amplification protocols and procedures for storing the amplified library.
Screening of the cDNA Library. To screen the library it must be placed on an appropriate host strain (e.g., Y1090 or Y1088 for λgtlO libraries, C600hf1A for λgtlO libraries). After plating the phage, plaques are transferred to nitrocellulose or nylon filters (See Ausubel et al., *supra* and Sambrook et al. *supra).* The filters are then probed with a³²P-labelled nick translated probe derived from rITF. The probe is preferentially generated using a portion of the region of rITF DNA coding for the trefoil structure (nucleotides 114 to 230 of SEQ ID NO. 1 which encode cyS32 to phe⁷¹ of SEQ ID NO. 2). This region is conserved between rITF, pS2 and PSP, and it is likely that this region is conserved between rITF and hITF. Once a plaque is identified several cycles of plaque purification are required to isolate a pure clone encoding hITF. A phage DNA isolation is performed and the cDNA insert can be subcloned into an appropriate vector for restriction mapping and sequencing. If the phage vector is Lambda ZAP® II, coinfection with helper phage allows rescue and recircularization of pBluescript SK⁻ phagemid vector (Stratagene, La Jolla, CA) harboring the cDNA; alternatively the phage clone is purified and the cDNA insert is subcloned into a vector suitable for restriction mapping and sequencing. If the clone does not contain the entire hITF gene (as assessed by homology to rITF and the presence of start and stop codons), the library can be rescreened with the original rITF probe or, preferably, with a probe generated from the hITF clone obtained. If none of the clones contain the intact gene, it can be reconstructed from clones which bear overlapping fragments of hITF.

### Direct Isolation of an hITF Probe by PCR

It is possible to isolate part of the hITF gene directly from the packaged library or cDNA. To isolate a portion of hITF directly from the packaged library, a pair of oligonucleotide primers and Taq polymerase are used to amplify the DNA corresponding to the hITF gene. The primers used would be approximately 15-20 nucleotides long and correspond in sequence to the 5'-most and 3'-most portions of the rITF coding sequence. Friedman et al. (in *PCR Protocols: A Guide to Methods and Applications,* Innis et al., eds., Academic Press, San Diego) describe a procedure for such amplification. Briefly, phage particles are disrupted by heating; Taq polymerase, primers (300 pmol of each), dNTPs, and Taq polymerase buffer are added; and the mixture is thermally cycled to amplify DNA. The amplified DNA is isolated by agarose gel electrophoresis. The ends of the fragment are prepared for ligation into an appropriate vector by making them flush with T4 polymerase and, if desired, adding linkers. Alternatively, a restriction site may be engineered into the fragment by using primers which have sequence added to their 5' ends which sequence will generate an appropriate sticky end when digested. For example the sequence: 5'-GGGCGGCCGC3' can be added to the 5' end of each primer. This sequence includes the NotI restriction site flanked at the 5' end by the sequence: GG. The additional nucleotides prevent the 5' ends from denaturing and interfering with subsequent restriction digestion with *NotI.* The gel purified DNA of the appropriate size is next cloned into a cloning vector for sequencing and restriction mapping. This clone will not have the entire hITF sequence, rather it will be a combination of hITF (the region between the sequences corresponding to the primers) and rITF (the 5' and 3' ends which correspond to the primer sequences). However, this DNA can be used to generate a labelled probe (produced by nick translation or random primer labelling) which, since it is the correct hITF sequence, can be used in a high stringency screening of the library from which the cDNA was originally isolated. In an alternative approach, cDNA can be used in the above procedure instead of a packaged library. This eliminates the steps of modifying the cDNA for insertion into a vector as well as cDNA packaging and library amplification. Ausubel et al. *supra* provides a protocol for amplification of a particular DNA fragment directly from cDNA and a protocol for amplification from poly(A)⁺ RNA.

### Identification of a Presumptive Human ITF clone

A nick translated probe derived from rITF cDNA (corresponding to nucleotides 1 to 431 of SEQ ID No. 1) was used for Northern blot analysis of poly(A)⁺ RNA derived from human intestinal mucosal scrapings. Probe hybridization and blot washing were carried out according to standard procedures. Probe (5 x 10⁵ cpm/ml hybridization buffer) was hybridized to the filter at 45°C in 5X SSC with 30% formamide. The filter was then washed at 60°C in 5X SSC with 40% formamide. Using this protocol a band was clearly visible after an overnight exposure of the filter with an intensifying screen. This result indicated that there is sufficient homology between rITF and hITF to allow the use of probes derived from the sequence of the rITF gene for identification of the hITF gene.

A human intestinal cDNA library was obtained from Clontech (Palo Alto, CA). Alternatively, a human intestinal cDNA library may be produced from mucosal scrapings as described above. Four oligonucleotide probes were selected for screening the library cDNA. Two of the probes correspond to sequences within the region of rITF encoding the trefoil and are referred to as internal probes (5'gtacattctgtctcttgcaga-3' and 5'-taaccctgctgctgctggtcctgg3'). The other two probes recognize sequences within rITF but outside of the trefoil encoding region and are referred to as external probes (5'-gtttgcgtgctgccatggaga-3' and 5'-ccgcaattagaacagccttgt-3'). These probes were tested for their utility by using them to screen the rat intestinal cDNA library described above. Each of the four probes could be used to identify a clone harboring all or part of the rITF gene. This result indicates that these probes may be used to screen the human intestinal library for the presence of hITF.

The internal probes were used as described above to amplify a DNA fragment from human colon library cDNA (Clontech, Palo Alto, CA). Linkers were added to the isolated DNA fragment which was then inserted into pBluescript phagemid vector (Stratagene, La Jolla, CA). The region of this clone corresponding to the sequence of human cDNA (i.e., not including the sequence corresponding to the internal probes) was used to make a radioactively labelled probe by random oligonucleotide-primed synthesis (Ausebel et al., *supra).* This probe was then used to screen the human colon cDNA library. This screening led to the identification of 29 clones. One of these clones (HuPCR-ITF) was nick-translated to generate a probe for Northern analysis of poly(A)⁺ RNA isolated from human intestinal mucosal scrapings. A single band of roughly the same size as the rat transcript (approximately 0.45 kD) was observed.

Northern analysis of poly(A)⁺ isolated from human tissues indicated that RNA corresponding to this probe was expressed in the small intestine and the large intestine but not in the stomach or the liver. These results indicate that the clone does not encode the human homolog of porcine PSP. Porcine PSP is expressed in porcine pancreas and is not significantly expressed in the small or large intestine. These results also distinguish the cloned gene from pS2 which is expressed in the stomach.

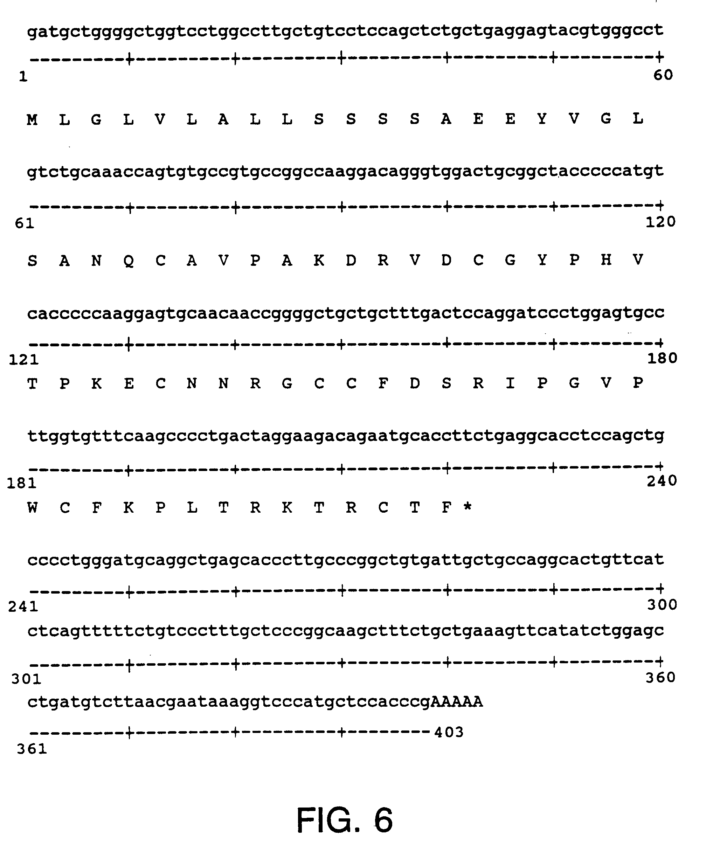

Figure 6 shows the nucleic acid sequence information for human ITF cDNA, along with the deduced amino acid sequence in one-letter code (SEQ ID NO: 3). This clone was obtained by the methods described above.

### Production of hITF

The isolated hITF gene can be cloned into a mammalian expression vector for protein expression. Appropriate vectors include pMAMneo (Clontech, Palo Alto, CA) which provides a RSV-LTR enhancer linked to a dexamethasone-inducible MMTV-LTR promoter, an SV40 origin of replication (allows replication in COS cells), a neomycin gene, and SV40 splicing and polyadenylation sites. This vector can be used to express the protein in COS cells, CHO cells, or mouse fibroblasts. The gene may also be cloned into a vector for expression in drosophila cells using the bacoluvirus expression system.

### Purification of Intestinal Trefoil Factor

Intestinal trefoil factor can be purified from intestinal mucosal scrapings of human, rats or any other species which expresses ITF (pigs and cows may provide a source of ITF). The purification procedure used for PSP will be useful for the purification of ITF since the proteins are likely to be homologous. Jorgensen et al. describes a method for purification of PSP (Regulatory Peptides 3:207, 1982). The preferred method is the second approach described by Jorgensen et al. *(supra).* This method involves chromatography of SP-Sephadex C-25 and QAE Sephadex A-25 columns (Sigma, St. Louis, MO) in acidic buffer.

### Anti-Intestinal Trefoil Factor Monoclonal Antibodies

Anti-intestinal trefoil factor monoclonal antibodies can be raised against synthetic peptides whose sequences are based on the deduced amino acid sequence of cloned hITF (SEQ ID NO: 3). Most commonly the peptide is based on the amino-or carboxy-terminal 10-20 amino acids of the protein of interest (here hITF). The peptide is usually chemically cross-linked to a carrier molecule such as bovine serum albumin or keyhole limpet hemocyanin. The peptide is selected with the goal of generating antibodies which will cross-react with the native hITF. Accordingly, the peptide should correspond to an antigenic region of the peptide of interest. This is accomplished by choosing a region of the protein which is (1) surface exposed, e.g., a hydrophobic region or (2) relatively flexible, e.g., a loop region or a β-turn region. In any case, if the peptide is to be coupled to a carrier, it must have an amino acid with a side chain capable of participating in the coupling reaction. See Hopp et al. *(Mol. Immunol.* 20:483, 1983; *J. Mol. Biol.* 157:105, 1982) for a discussion of the issues involved in the selection of antigenic peptides. A second consideration is the presence of a protein homologous to hITF in the animal to be immunized. If such a protein exists, it is important to select a region of hITF which is not highly homologous to that homolog.

For hITF, peptides that correspond to the amino-terminal or carboxy-terminal 15 amino acids are likely to be less homologous across species and exposed to the surface (and thus antigenic). Thus they are preferred for the production of monoclonal antibodies. Purified hITF can also be used for the generation of antibodies.

In practice ITF may be administered orally, intravenously, or intraperitoneally for treatment of peptic ulcer diseases, inflammatory bowel diseases, and for protection of the intestinal tract from injury caused by bacterial infection, radiation injury or other insults. The mode of administration, dosage, and formulation of ITF depends upon the condition being treated.

Other embodiments are feasible. For example, ITF may be used to produce monoclonal antibodies for the detection of ITF in intestinal tissue or blood serum by means of an indirect immunoassay. ITF may be detectably labelled and used in an *in situ* hybridization assay for the detection of ITF binding sites. Labels may include, but are not limited to, florescein or a radioactive ligand.

ITF may be used to protect and stabilize other proteins. This protection is accomplished by forming a hybrid molecule in which all or part of ITF is fused to either the carboxy-terminus or the amino-terminus (or both) of the protein of interest. Because ITF is resistant to degradation in the digestive system, it will protect the protein of interest from such degradation. As a consequence, the protein of interest is likely to remain active in the digestive system and/or will be more readily absorbed in an intact form.

## Claims

1. A purified nucleic acid encoding an intestinal trefoil factor comprising a polypeptide that is at least 70% homologous to SEQ ID NO: 2.

2. A purified nucleic acid according to Claim 1, wherein said polypeptide is a naturally occurring peptide with a trefoil configuration derivable from a mammalian intestine.

3. A purified nucleic acid according to Claim 2, wherein said mammalian intestine is a human intestine.

4. A purified nucleic acid according to Claim 2, wherein said mammalian intestine is a rat intestine.

5. A purified nucleic acid according to Claim 2, wherein said mammalian intestine comprises a cow intestine, or a pig intestine.

6. A cell comprising a vector encoding an intestinal trefoil factor comprising a polypeptide that is at least 70% homologous to SEQ ID NO: 2.

7. A substantially pure intestinal trefoil factor comprising a polypeptide that is at least 70% homologous to SEQ ID NO: 2, and is optionally detectably labelled.

8. A substantially pure intestinal trefoil factor according to Claim 7, wherein said polypeptide is a naturally occurring peptide with a trefoil configuration derivable from a human intestine.

9. A therapeutic composition comprising substantially pure intestinal trefoil factor according to Claim 7, and a pharmacologically acceptable carrier.

10. A monoclonal antibody which forms an immune complex with a substantially pure intestinal trefoil factor according to Claim 7, which antibody is optionally detectably labelled.

11. A method for detecting a human intestinal trefoil factor comprising a polypeptide which is at least 70% homologous to SEQ ID NO: 2 in a biological sample obtained from a human patient, comprising
contacting said sample with a monoclonal antibody according to Claim 10, and
detecting immune complexes formed with said monoclonal antibody.

12. A method according to Claim 11, wherein said biological sample is an intestinal mucosal scraping.

13. A method according to Claim 11, wherein said biological sample is a serum sample.

14. A method for detecting binding sites for an intestinal trefoil factor comprising a polypeptide which is at least 70% homologous to SEQ ID NO: 2 in a biological sample obtained from a patient comprising
contacting said sample with a substantially pure intestinal trefoil factor according to Claim 7 and
detecting said factor bound to said biological sample as an indication of the presence of said binding sites in said sample.

15. Isolated DNA comprising a sequence encoding an intestinal trefoil factor comprising a polypeptide that is at least 70% homologous to SEQ ID NO: 2.

16. Isolated DNA according to Claim 15, wherein said polypeptide is a naturally occurring peptide with a trefoil configuration derivable from a mammalian intestine.

17. Isolated DNA according to Claim 16, wherein said mammal is a human.

18. Isolated DNA according to Claim 16, wherein said mammal is a rat.

19. Isolated DNA according to Claim 16, wherein said mammal comprises a cow, or a pig.

20. A vector comprising nucleic acid according to Claim 1 or isolated DNA according to Claim 15.

21. A polypeptide according to Claim 7, for use in therapy to treat digestive disorders.

22. Use of a polypeptide according to Claim 7, for the manufacture of a medicament for use in treating digestive disorders.

23. A purified nucleic acid comprising the sequence of SEQ ID NO: 1.

24. A substantially pure polypeptide comprising the sequence of SEQ ID NO: 2.

25. A purified nucleic acid encoding a human intestinal trefoil factor, the nucleic acid producible by a method comprising the steps of:
(a) providing a human intestinal cDNA library;
(b) providing a pair of oligonucleotide primers having sequences corresponding to opposite strands of a trefoil encoding region of rat intestinal trefoil factor nucleic acid, the rat nucleic acid having a sequence SEQ ID: 1;
(c) screening the cDNA library for nucleic acid sequences which are amplified by the oligonucleotide pairs in a polymerase chain reaction; and
(d) selecting from the cDNA library those sequences which correspond to the amplified nucleic acid sequences and which show a pattern of expression in the small and large intestine, but not in stomach or liver.

## Patentansprüche

1. Gereinigte Nukleinsäure, die für einen intestinalen Kleeblattfaktor codiert, der ein Polypeptid umfasst, das mindestens zu 70% zu SEQ ID NO: 2 homolog ist.

2. Gereinigte Nukleinsäure nach Anspruch 1, wobei das Polypeptid ein natürlich auftretendes Peptid mit einer Kleeblatt-Konfiguration ist, das aus einem Säugerdarm erhältlich ist.

3. Gereinigte Nukleinsäure nach Anspruch 2, wobei der Säugerdarm ein Menschendarm ist.

4. Gereinigte Nukleinsäure nach Anspruch 2, wobei der Säugerdarm ein Rattendarm ist.

5. Gereinigte Nukleinsäure nach Anspruch 2, wobei der Säugerdarm einen Kuhdarm oder einen Schweinedarm umfasst.

6. Zelle, die einen Vektor umfasst, der für einen intestinalen Kleeblattfaktor codiert, der ein Polypeptid umfasst, das mindestens zu 70% zu SEQ ID NO: 2 homolog ist.

7. Im Wesentlichen reiner intestinaler Kleeblattfaktor, der ein Polypeptid umfasst, das mindestens zu 70% zu SEQ ID NO: 2 homolog ist und das gegebenenfalls nachweisbar markiert ist.

8. Im Wesentlichen reiner intestinaler Kleeblattfaktor nach Anspruch 7, wobei das Polypeptid ein natürlich auftretendes Peptid mit einer Kleeblattkonfiguration ist, das aus einem Menschendarm erhältlich ist.

9. Therapeutische Zusammensetzung, die im Wesentlichen reinen intestinalen Kleeblattfaktor gemäß Anspruch 7 und einen pharmakologisch annehmbaren Träger umfasst.

10. Monoklonaler Antikörper, der mit einem im Wesentlichen reinen intestinalen Kleeblattfaktor nach Anspruch 7 einen Immunkomplex bildet, wobei der Antikörper gegebenenfalls nachweisbar markiert ist.

11. Verfahren zum Nachweis eines humanen intestinalen Kleeblattfaktors, der ein Polypeptid umfasst, das mindestens zu 70% zu SEQ ID NO: 2 identisch ist, in einer biologischen Probe, die von einem Menschenpatienten erhalten wird, umfassend:
- In Kontakt bringen dieser Probe mit einem monoklonalen Antikörper gemäß Anspruch 10 und
- Nachweisen von Immunkomplexen, die mit dem monoklonalen Antikörper gebildet wurden.

12. Verfahren nach Anspruch 11, wobei die biologische Probe ein Darmschleimhautabstrich ist.

13. Verfahren nach Anspruch 11, wobei die biologische Probe eine Serumprobe ist.

14. Verfahren zum Nachweis von Bindungsstellen für einen intestinalen Kleeblattfaktor, der ein Polypeptid umfasst, das mindestens zu 70% homolog zu SEQ ID NO: 2 ist, in einer biologischen Probe, die von einem Patienten erhalten wird, umfassend:
- In-Kontakt-bringen der Probe mit einem im Wesentlichen reinen intestinalen Kleeblattfaktor nach Anspruch 7 und
- Nachweisen des Faktors, der an die biologische Probe gebunden ist, als Hinweis für das Vorliegen der Bindungsstellen in der Probe.

15. Isolierte DNA, die eine Sequenz umfasst, welche für einen intestinalen Kleeblattfaktor codiert, der ein Polypeptid umfasst, das mindestens zu 70% zu SEQ ID NO: 2 homolog ist.

16. Isolierte DNA nach Anspruch 15, wobei das Polypeptid ein natürlich auftretendes Peptid mit einer Kleeblattkonfiguration ist, das aus einem Säugerdarm erhältlich ist.

17. Isolierte DNA nach Anspruch 16, wobei der Säuger ein Mensch ist.

18. Isolierte DNA nach Anspruch 16, wobei der Säuger eine Ratte ist.

19. Isolierte DNA nach Anspruch 16, wobei der Säuger eine Kuh oder ein Schwein umfasst.

20. Vektor, der Nukleinsäure nach Anspruch 1 oder isolierte DNA nach Anspruch 15 umfasst.

21. Polypeptid nach Anspruch 7 zur Verwendung in der Therapie zur Behandlung von Verdauungskrankheiten.

22. Verwendung eines Polypeptids nach Anspruch 7 zur Herstellung eines Medikaments zur Verwendung bei der Behandlung von Verdauungskrankheiten.

23. Gereinigte Nukleinsäure, die die Sequenz von SEQ ID NO: 1 umfasst.

24. Im Wesentlichen reines Polypeptid, das die SEQ ID NO: 2 umfasst.

25. Gereinigte Nukleinsäure, die für einen humanen intestinalen Kleeblattfaktor codiert, wobei die Nukleinsäure nach einem Verfahren produzierbar ist, das die folgenden Schritte umfasst:
(a) Bereitstellen einer humanen intestinalen cDNA-Bibliothek;
(b) Bereitstellen eines Paars Oligonukleotidprimer mit Sequenzen, die den entgegengesetzten Strängen einer Kleeblatt codierenden Region der Nukleinsäure von intestinalem Ratten-Kleeblattfaktor entsprechen, wobei die Rattennukleinsäure eine Sequenz SEQ ID NO: 1 hat;
(c) Durchmustern der cDNA-Bibliothek nach Nukleinsäuresequenzen, die in einer Polymerasekettenreaktion durch die Oligonukleotidpaare amplifiziert werden;
(d) Selektieren aus der cDNA-Bibliothek, die Sequenzen, die den amplifizierten Nukleinsäuresequenzen entsprechen und die ein Expressionsmuster im Dünn- und Dick-Darm, nicht aber im Magen oder der Leber zeigen.

## Revendications

1. Acide nucléique purifié codant pour un facteur intestinal en feuille de trèfle comprenant un polypeptide ayant une homologie d'au moins 70 % avec SEQ ID N° 2.

2. Acide nucléique purifié selon la revendication 1, ledit polypeptide étant un peptide naturel à configuration en feuille de trèfle, pouvant être obtenu à partir d'un intestin de mammifère.

3. Acide nucléique purifié selon la revendication 2, ledit intestin de mammifère étant un intestin humain.

4. Acide nucléique purifié selon la revendication 2, ledit intestin de mammifère étant un intestin de rat.

5. Acide nucléique purifié selon la revendication 2, ledit intestin de mammifère comprenant un intestin de vache ou de porc.

6. Cellule comprenant un vecteur codant pour un facteur intestinal en feuille de trèfle comprenant un polypeptide ayant une homologie d'au moins 70 % avec SEQ ID N° 2.

7. Facteur intestinal en feuille de trèfle essentiellement pur comprenant un polypeptide qui a une homologie d'au moins 70 % avec SEQ ID N° 2, et qui est éventuellement marqué par un marqueur décelable.

8. Facteur intestinal en feuille de trèfle essentiellement pur selon la revendication 7, ledit polypeptide étant un peptide naturel à configuration en feuille de trèfle, pouvant être obtenu à partir d'un intestin humain.

9. Composition thérapeutique comprenant un facteur intestinal en feuille de trèfle essentiellement pur selon la revendication 7 et un support pharmacologiquement acceptable.

10. Anticorps monoclonal qui forme un complexe immun avec un facteur intestinal en feuille de trèfle essentiellement pur selon la revendication 7, cet anticorps étant éventuellement marqué avec un marqueur décelable.

11. Procédé de détection d'un facteur intestinal humain en feuille de trèfle comprenant un polypeptide ayant une homologie d'au moins 70 % avec SEQ ID N° 2 dans un échantillon biologique prélevé chez un patient humain, comprenant
la mise en contact dudit échantillon avec un anticorps monoclonal selon la revendication 10, et
la détection de complexes immuns formés avec ledit anticorps monoclonal.

12. Procédé selon la revendication 11, dans lequel ledit échantillon biologique est un produit de grattage de la muqueuse intestinale.

13. Procédé selon la revendication 11, dans lequel ledit échantillon biologique est un échantillon de sérum.

14. Procédé de détection de sites de liaison pour un facteur intestinal en feuille de trèfle comprenant un polypeptide ayant une homologie d'au moins 70 % avec SEQ ID N° 2 dans un échantillon biologique prélevé chez un patient, comprenant
la mise en contact dudit échantillon avec un facteur intestinal en feuille de trèfle essentiellement pur selon la revendication 7 et
la détection dudit facteur lié audit échantillon biologique en tant qu'indication de la présence desdits sites de liaison dans ledit échantillon.

15. ADN isolé comprenant une séquence codant pour un facteur intestinal en feuille de trèfle comprenant un polypeptide ayant une homologie d'au moins 70 % avec SEQ ID N° 2.

16. ADN isolé selon la revendication 15, ledit polypeptide étant un peptide naturel à configuration en feuille de trèfle pouvant être obtenu à partir d'un intestin de mammifère.

17. ADN isolé selon la revendication 16, ledit mammifère étant un être humain.

18. ADN isolé selon la revendication 16, ledit mammifère étant un rat.

19. ADN isolé selon la revendication 16, ledit mammifère comprenant une vache ou un porc.

20. Vecteur comprenant un acide nucléique selon la revendication 1 ou un ADN isolé selon la revendication 15.

21. Polypeptide selon la revendication 7, à utiliser dans une thérapie pour le traitement de troubles digestifs.

22. Utilisation d'un polypeptide selon la revendication 7 pour la préparation d'un médicament à utiliser dans le traitement de troubles digestifs.

23. Acide nucléique purifié comprenant la séquence de SEQ ID N° 1.

24. Polypeptide essentiellement pur comprenant la séquence de SEQ ID N° 2.

25. Acide nucléique purifié codant pour un facteur intestinal humain en feuille de trèfle, l'acide nucléique pouvant être produit par un procédé comprenant les étapes selon lesquelles:
(a) on fournit une banque d'ADNc intestinal humain;
(b) on fournit une paire d'amorces oligonucléotidiques ayant des séquences correspondant aux brins opposés d'une région codant pour une feuille de trèfle d'un acide nucléique de facteur intestinal de rat en feuille de trèfle, l'acide nucléique de rat ayant la séquence SEQ ID N° 1;
(c) on crible la banque d'ADNc pour rechercher les séquences d'acides nucléiques qui sont amplifiées par les paires d'oligonucléotides dans une amplification en chaîne par polymérase; et
(d) on sélectionne dans la banque d'ADNc les séquences qui correspondent aux séquences d'acides nucléiques amplifiées et qui présentent un profil d'expression dans le gros intestin et l'intestin grêle, mais non dans l'estomac ni dans le foie.
